# EUROPÄISCHE PATENTSCHRIFT

(11) **EP 3 260 084 B1**
(45) Veröffentlichungstag und Bekanntmachung des Hinweises auf die Patenterteilung: **07.11.2018**
(21) Anmeldenummer: 17176863.3
(22) Anmeldetag: 20.06.2017
(51) Int. Cl.: A61F 2/24, A61F 2/966, A61M 25/00

(54) **EINFÜHRKATHETER UND KATHETERANORDNUNG**
DELIVERY CATHETER AND CATHETER ARRANGEMENT
CATHÉTER DE LARGAGE ET SYSTÈME DE CATHÉTER

(30) Priorität: 21.06.2016 DE 102016111323
(43) Veröffentlichungstag der Anmeldung: 27.12.2017
(73) Patentinhaber: Biotronik AG, 8180 Bülach (CH)
(72) Erfinder: Keller, Mark, 5000 Aarau (CH); Hepke, Markus, 8006 Zürich (CH)
(74) Vertreter: Galander, Marcus

(56) Entgegenhaltungen:
- WO-A1-2012/009006
- WO-A2-2012/023981
- DE-T2- 69 635 676
- US-A1- 2009 264 859
- US-A1- 2013 079 872

## Beschreibung

Die Erfindung betrifft einen Einführkatheter zum Einführen eines Implantats an einem Zielort in einem Gefäß oder Organ eines Wirbeltiers, wobei der Einführkatheter einen Innenschaft, der zum Tragen des Implantats während des Einführens und zum Freisetzen desselben am Zielort ausgebildet ist und der eine atraumatische distale Katheterspitze aufweist, und einen den Innenschaft umgebenden und diesem gegenüber verschieblichen Außenschaft zum Verschieben des Implantats an den Zielort aufweist, wobei der Außenschaft an seinem distalen Ende eine Implantatkapsel zur umhüllenden Aufnahme des Implantats während des Einführens trägt.

Minimal invasive chirurgische Eingriffe gewinnen seit Jahren ständig an Bedeutung und sind beispielsweise zur Behandlung von Stenosen unverzichtbar. In letzter Zeit werden sie auch verstärkt bei der Implantation künstlicher Herzklappen eingesetzt. Geeignete Einführkatheter sind in großer konstruktiver Vielfalt bekannt und Gegenstand ständiger Weiterentwicklung. In den letzten Jahren wurde hier bei der Weiterentwicklung zu Kathetern besonderes Augenmerk geschenkt, die nicht nur das Setzen, sondern auch das Zurückziehen bzw. eine Positionierung von kardiovaskulären Implantaten ermöglichen.

Derartige Einführkatheter bestehen im Wesentlichen aus einem ersten, inneren Katheterschaft, auf dessen distalem Ende das Implantat angeordnet ist. Das Implantat und der erste, innere Katheterschaft sind von einem zweiten, äußeren Katheterschaft umgeben. Der distale Bereich des zweiten äußeren Katheterschafts, der das Implantat umgibt, wird häufig als Implantatkapsel, oder gelegentlich als Katheterhülle bezeichnet. Die Implantatkapsel kann dabei aus dem gleichen Material wie der zweite äußere Katheterschaft oder aus einem anderen Material bestehen, welches mit dem zweiten äußeren Katheterschaft verbunden wird. Hier wird unter der Positionsbezeichnung "proximal" ein näher zum Operateur liegender Teil des Einführkatheters bezeichnet. "Distal" bezeichnet entsprechend einen Teil des Einführkatheters, der sich weiter entfernt vom Operateur befindet.

Häufig sind die Implantate unter Einsatz von Formgedächtnismaterial konstruiert. In diesen Fällen werden die Implantate während des Einführens an den Implantationsort durch die sie umgebende Katheterhülle in ihrer komprimierten Form gehalten. Durch Verschieben der Katheterhülle, beispielsweise nach proximal, verschwindet die Rückhaltekraft, die die Katheterhülle auf das Implantat ausübt, und das Implantat expandiert.

Vor kurzem wurden Lösungen vorgeschlagen, die ein Zurückführen ("resheathing") eines bereits freigesetzten Implantats in den Einführkatheter, also speziell in die Implantatkapsel, ermöglichen sollen. Derartige Lösungen werden von den Operateuren stark begrüßt, weil sie Korrekturen während des Einführvorganges ermöglichen und somit helfen, den Implantationsvorgang mit dem bestmöglichen Ergebnis abzuschließen. Speziell beim Zurückziehen von Herzklappenstents in die Implantatkapsel bzw., präziser formuliert, beim Wiederaufstülpen des distalen Katheterendes über den Stent, treten relativ hohe Reaktionskräfte auf. Diese führen zu komplexen Problemen wie beispielsweise in der Beschreibung des Standes der Technik in der US 2011/0098804 A1 beschrieben und mit dem Versuch einer (teilweisen) Lösung verknüpft.

Es ist bekannt, die Katheterspitze aus einem weichen/atraumatischen Material zu fertigen, um Verletzungen der Gefäßwände beim Vorschieben der Kathetereinrichtung möglichst zu vermeiden. Derartige Ausführungen bieten ausreichend Schutz der empfindlichen Gefäßinnenwand und der natürlichen Aortenklappe, wenn diese mit einem Aortenklappenersatzkatheter passiert werden. Bei sehr starken Biegungen des Übergangsbereichs zwischen distaler Katheterspitze und anschließendem Außenschlauch (Kapsel, die das Implantat beinhaltet) kann es dazu kommen, dass Kanten freistehen, die die Gefäßinnenwand oder die natürliche Aortenklappe verletzen können. Besonders bei Systemen der oben erwähnten Art, die ein teilweise freigesetztes Implantat wiedereinfangen können werden die Katheterkomponenten stark beansprucht was zu einer noch ausgeprägteren Kantenbildung führen kann. Die Ursache für diese ausgeprägte Kantenbildung ist die trichterförmige Aufweitung der Kapsel durch die Zurückführung über das teilweise freigesetzte Implantat. Es entsteht eine vergrößerte Umfangskante am distalen Katheterende.

Der Erfindung liegt daher die Aufgabe zu Grunde, einen verbesserten Einführkatheter sowie eine entsprechende Katheteranordnung anzugeben, die Verletzung der Gefäßinnenwand oder der natürlichen Aortenklappe im Gebrauch, und zwar speziell beim oder nach einem Resheathen, weitgehend ausschließen.

Diese Aufgabe wird durch einen Einführkatheter mit den Merkmalen des Anspruchs 1 gelöst. Zweckmäßige Fortbildungen des Erfindungsgedankens sind Gegenstand der abhängigen Ansprüche.

Die Erfindung schließt die Überlegung ein, am distalen Ende der Implantatkapsel ein temporär wirksames Schutzelement bzw. einen "Puffer" mit ausreichend großer radialer Erstreckung vorzusehen, um das gefährliche Freistehen des Endes der Implantatkapsel über die Katheterspitze des Einführkatheters situationsbezogen zu vermeiden. Sie schließt weiter den Gedanken ein, dies durch eine temporäre Vergrößerung der radialen Erstreckung der weichen Katheterspitze zu erreichen. Weiterhin gehört zur Erfindung der Gedanke, diese temporäre Vergrößerung des Spitzenradius durch ein Betätigungselement vorzunehmen, das in bestimmten Benutzungssituationen, jedoch im Rahmen der üblichen Handhabungen und ohne Verkomplizierung des Bedienvorganges für den Operateur, seine Wirkung entfaltet. Schließlich umfasst die Erfindung den Gedanken, als solches Betätigungselement ein in den Außenschaft eingelegtes Stempelelement vorzusehen, das bei einem Vorschieben des Außenschaftes relativ zum Innenschaft über einen vorbestimmten Punkt hinaus an seinem proximalen Ende mit der axial einwirkenden Druckkraft beaufschlagt wird. Es überträgt diese Druckkraft auf das proximale Ende der Katheterspitze, wodurch deren elastische Aufweitung bewirkt wird.

In Folge dessen, dass die atraumatische Katheterspitze im aufgeweiteten Zustand einen größeren Durchmesser hat als das aufgeweitete distale Ende der Implantatkapsel, stellt dieses nun keine exponierte Kante mehr dar, an der sich die Implantatkapsel an Ablagerungen oder anatomischen Strukturen im Gefäß bzw. Herzen des Patienten verhaken und hierdurch Verletzungen auslösen kann. Durch diese Lösung besteht während der Aortenklappen Implantation ein geringeres Risiko des Ablösens von Ablagerungen (Plaque) und der Verletzung von anatomischen Strukturen wie Gefäßinnenwand und natürlicher Aortenklappe durch das Kathetersystem. Sie verringert somit die Wahrscheinlichkeit einer Embolie. Weiter begünstigt diese Lösung ein einfacheres Recrossing der nativen Klappe, da sich das System nicht mehr mit den exponierten Kanten der distal aufgeweiteten Kapsel verfangen kann.

Die Handhabung des erfindungsgemäßen Einführkatheters, unter Beachtung charakteristischer konstruktiver Aspekte desselben, lässt sich wie folgt beschreiben:
Um die partiell freigesetzte Prothese wieder einzufangen, wird die Implantatkapsel nach distal bewegt. Die Kapsel ist am Außenschaft des Katheters befestigt. Dieser Außenschaft wird über den beweglichen Teil des Griffes angetrieben und vorwärts (nach distal) bewegt. Dieser Außenschaft wiederum stößt während des Resheathing-Vorganges ab einem bestimmten Punkt (overstroke) auf das Stempelelement. Dieses ist so konstruiert, dass es durch den Prothesenkonnektor hindurchgeführt ist. Weiter ist es so angeordnet, dass es durch die aufgekrimmte Prothese in ihrer axialen Bewegung nicht behindert wird.

Dieses Stempelelement wird nun nach dem Resheathing zur atraumatischen Katheterspitze hin bewegt und herangedrückt. Der axiale Druck des Stempelelementes auf die Spitze führt dazu, dass die Spitze sich in radialer Richtung elastisch ausdehnt bzw. verformt. Dieses Ausdehnen in radialer Richtung wird bewerkstelligt, indem die Spitze an ihrem distalen Ende mit dem Innenschaft des Katheters starr verbunden ist. Wenn die Spitze mit axialem Druck beaufschlagt wird, wird sie axial zusammengestaucht, und sie vergrößert sich als Folge daraus in radialer Richtung.

Während des abermaligen Freisetz-Prozesses der Prothese wird sich die Spitze wieder in ihre ursprüngliche Form zurückfinden, da die Druckspannung nicht mehr auf sie einwirkt und die Rückstellkräfte bewerkstelligen, dass sie sich wieder axial und radial entspannt.

Die Vorteile der vorliegenden Erfindung kommen insbesondere zum Tragen, wenn das Implantat als Herzklappenprothese ausgeführt ist. Eine derartige Herzklappenprothese umfasst im Wesentlichen ein selbstexpandierendes Grundgerüst (Stent) und eine darin angeordnete und daran befestigte Klappenanordnung. Das Grundgerüst ist zweckmäßigerweise mit Mitteln ausgestattet, die mit einem entsprechenden Gegenstück auf dem Innenschaft (Implantat-Konnektor) eine axiale Fixierung des Stents auf dem Innenschaft solange gewährleisten, bis die Herzklappenprothese vollständig durch Rückzug der Implantatkapsel freigesetzt ist. Die Herzklappenprothese ist entsprechend bevorzug lösbar mit dem Implantat-Konnektor verbunden.

Bevorzugt ist das Stempelelement mit einem geeigneten Betätigungselement, vorzugsweise am Handgriff des Katheters, verbunden, welches nach dem Resheating über einen vorgegebenen Punkt hinausbewegt wird und die Bewegung des Stempelelements zur Katheterspitze hin auslöst.

In einer bevorzugten Ausführung der Erfindung ist die Katheterspitze als mindestens im aufweitbaren proximalen Abschnitt weich-elastisches Kunststoff-Formteil ausgebildet. Weiter bevorzugt ist eine insgesamt, also über die gesamte Länge, weich-elastische Ausführung der Katheterspitze; jedoch ist durch die distale feste Verbindung mit dem Innenschaft in jedem Falle eine gewisse Versteifung im distalen Bereich gegeben.

In einer weiteren Ausführung ist die Katheterspitze am proximalen Ende nicht am Innenschaft fixiert, sondern beim Aufweiten gegenüber dem Innenschaft gleitbar ausgeführt. Grundsätzlich ist dieses Merkmal für die Ausführung der Erfindung nicht zwingend; die erfindungsgemäße Aufweitung der Katheterspitze kann grundsätzlich auch erreicht werden, wenn das proximale Ende der Katheterspitze festgelegt ist. Die Verschieblichkeit bietet aber vorteilhafte Realisierungsmöglichkeiten der Aufweitung.

Im Zusammenhang hiermit steht eine Ausführung, bei der in das proximale Ende der Katheterspitze eine die Gleitfähigkeit gegenüber dem Innenschaft erhöhende und die Kraftübertragung vom Stempelelement auf das proximale Ende der Katheterspitze verbessernde Gleitbüchse eingelegt ist. Ein solches zusätzliches Element an der Katheterspitze, welches bevorzugt aus etwas härterem Kunststoff als die Spitze selbst gefertigt ist, macht die Fertigung des Einführkatheters zwar etwas aufwändiger, verbessert aber die Funktionsfähigkeit.

Eine weitere Optimierung der Funktionsfähigkeit wird dadurch erreicht, dass die Katheterspitze über den größeren Teil ihrer Längserstreckung an ihrem gesamten Innenumfang mit dem Außenumfang des Innenschaftes stoffschlüssig verbunden, insbesondere verklebt oder verschweißt, ist. Diese Maßnahme wirkt zum einen stabilitätserhöhend und dient zum anderen zur Vermeidung unkontrollierter Verformungen über den Längsverlauf der Spitze. Nach den Untersuchungen der Erfinder ist es zweckmäßig, wenn der Durchmesser der Katheterspitze an deren proximalem Ende im unaufgeweiteten Zustand im Wesentlichen gleich dem Durchmesser der Implantatkapsel ist und der größte Durchmesser der Katheterspitze im aufgeweiteten Zustand im Bereich zwischen dem 1,05-fachen und dem 1,4-fachen, insbesondere dem 1,1-fachen und 1,35-fachen, besonders bevorzugt dem 1,25-fachen und 1,3-fachen, Durchmesser) der Implantatkapsel liegt. Dies gewährleistet in allen kritischen Anwendungssituationen einen hinreichenden Schutz der benachbarten Gefäßwände vor dem aufgeweiteten distalen Ende der Implantatkapsel.

In einer weiteren Ausführung der Erfindung ist das Stempelelement als in seinem distalen Bereich spiralig geschnittenes Metallrohr ausgebildet, an dessen distalem Ende ein Ringflansch zur Vergrößerung der Kontaktfläche mit der Katheterspitze ausgebildet ist. Diese Ausführung sichert zum einen die notwendige Drucksteifigkeit des Stempelelements, zur zuverlässigen Ausführung seiner Funktion und bietet andererseits die geforderte Biegsamkeit, um die Flexibilität des Einführkatheters beim Passieren stark gewundener Gefäßabschnitte zu erhalten.

In Anlehnung an bekannte Konstruktionen des Einführkatheters ist in einer weiteren Ausführung am Innenschaft der Implantatkapsel ein Implantat-Konnektor fest angebracht. Dieser weist hier Führungsmittel zum Führen des Stempelelementes bei deren axialer Verschiebung auf. In einer Fortbildung dieser Ausführung sind die Führungsmittel am Implantat-Konnektor als Durchgangslöcher oder Führungskanäle durch die Wandung des Implantat-Konnektors ausgebildet. Das Stempelelement ist in seinem proximalen Bereich streifig geschnitten, derart, dass die Streifen des Stempelelements durch die Durchgangslöcher des Implantat-Konnektors gefädelt sind. Die Streifen des Stempelelements sind an dessen proximalem Ende in einem Verbindungsring gefasst, der ein umfangsmäßig geschlossenes proximales Ende des Stempelelements definiert.

In einer weiteren Ausführung ist der Außenschaft in seinem Verbindungsbereich mit der Implantatkapsel derart aufgeweitet, dass eine ringförmige distale Stirnfläche gebildet ist, an die das proximale Ende des Stempelelements anstößt. Die distale Stirnfläche am Außenschaft stellt also selbst ebenfalls eine Art Stempel dar, mit dem das biegsame Stempelelement seinerseits nach distal gedrückt wird. Eine sinnreiche Verknüpfung mit der vorgenannten Ausführung sieht dann so aus, dass das proximale Ende des die Metallstreifen des Stempelelements zusammenfassenden Verbindungsringes an die distale ringförmige Stirnfläche des Außenschaftes anstößt.

Vorzugsweise weist die Katheterspitze einen proximalen Hohlraum auf. Bevorzugt umschließt der Hohlraum den Innenschaft. Ein Hohlraum im proximalen Bereich der Katheterspitze bzw. am proximalen Ende der Katheterspitze ist eine einfache Möglichkeit, die Katheterspitze axial komprimierbar und gleichzeitig radial expandierbar auszugestalten. Das Stempelelement drückt gegen das proximale Ende des Hohlraums, welcher sich radial ausdehnt und axial komprimiert wird. Die radiale Ausdehnung des Hohlraums wird durch die Fixierung des distalen Bereiches der Katheterspitze auf dem Innenschaft quasi erzwungen.

Unter einem Hohlraum wird in dieser Ausgestaltung eine Kavität verstanden. Der Hohlraum kann dabei gegenüber dem Innenschaft geöffnet (Halbschale oder ähnliches) oder geschlossen sein (Hohlkugel oder ähnliches mit einer Öffnung für den Innenschaft).

Vorteile und Zweckmäßigkeiten der Erfindung ergeben sich im Übrigen aus der nachfolgenden Beschreibung eines Ausführungsbeispiels anhand der Figuren. Von diesen zeigen:
- Fig. 1A bis 1D: Darstellungen des distalen Endbereiches einer Ausführungsform des erfindungsgemäßen Einführkatheters in Art einer Längsschnittdarstellung, in verschiedenen Phasen des Gebrauchs, und
- Fig. 2A und 2B: weiter vergrößerte Detailansichten der Katheterspitze und des distalen Teils des Stempelelementes in zwei Ausgestaltungen der in Fig. 1A bis 1D gezeigten Ausführungsform.

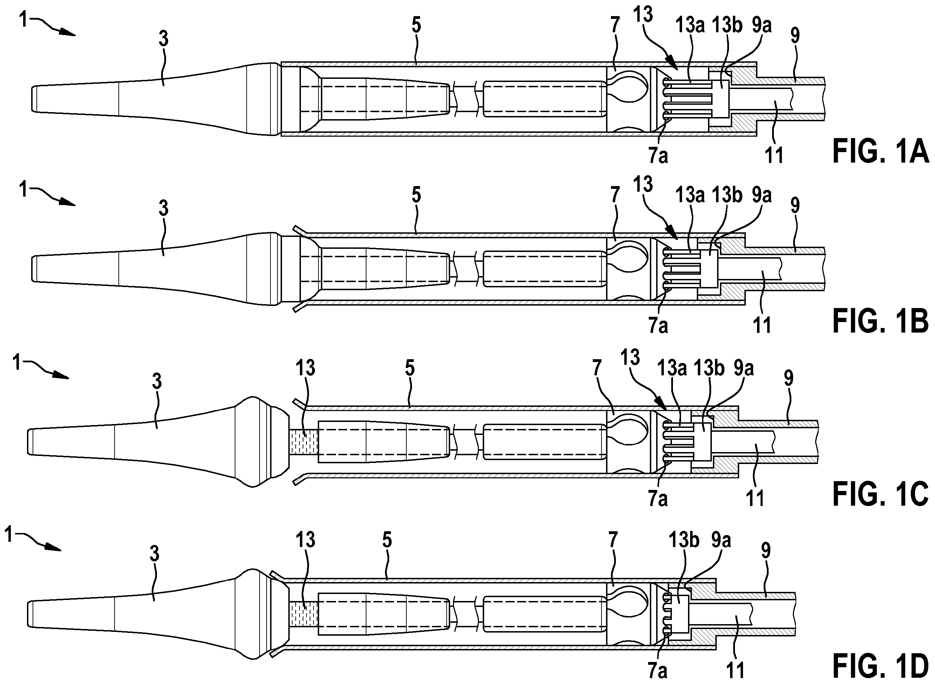

Die Figuren 1A bis 1D zeigen den distalen Endbereich eines Einführkatheters 1 in einer schematischen Darstellung in Art einer Längsschnittdarstellung, und zwar vom distalen Ende einer Katheterspitze 3 über eine Implantatkapsel 5 mit Implantat-Konnektor 7 bis zum distalen Endbereich eines Außenschaftes 9, wobei teilweise auch der Verlauf eines Innenschaftes 11 schematisch gezeigt ist. Der Einführkatheter 1 dient dabei dem Freisetzen einer Herzklappenprothese (nicht dargestellt) umfassend ein selbstexpandierendes Grundgerüst und eine Klappenanordnung. Fig. 1A zeigt einen Ausganszustand des Einführkatheters 1, bei dem die Implantatkapsel 5 noch unverformt ist, und Fig. 1B zeigt den Zustand nach der teilweisen Freisetzung einer Prothese bzw. eines Implantats und einem nachfolgenden Resheathing (Wiedereinfangen des Implantats), welches eine gewissermaßen trichterförmige Aufweitung des distalen Endes der Implantatkapsel bewirkt hat. Ein derartiger Zustand würde sich bei einem Katheter nach dem Stand der Technik ergeben.

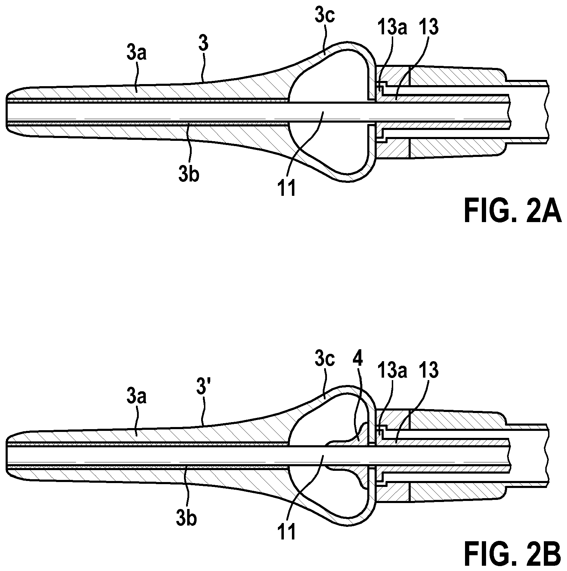

Fig. 1C und 1D zeigen Handhabungsphasen der Nutzung der erfindungsgemäßen Konstruktion, wobei zu deren Bestandteilen teilweise auf Fig. 2A und 2B zu verweisen ist. Der Außenschaft 9 wird hierbei während eines (weiteren) Resheathing-Vorganges nach distal (in der Figur nach links) bewegt und drückt dabei vor mittels des Anstoß-Kontaktes zwischen seiner distalen ringförmigen Stirnfläche 9a und dem proximalen Ende eines langgestreckten Stempelelementes 13 jenes Stempelelement, das sich im Wesentlichen über die gesamte Länge der Implantatkapsel 5 erstreckt (jedoch in Fig. 1A - 1D nicht in seiner ganzen Erstreckung zu erkennen ist), ebenfalls nach distal.

Das distale Ende des Stempelelementes, das in Fig. 2A und 2B zu sehen ist, stößt hierbei an das proximale Ende der Katheterspitze 3 (deren spezieller Aufbau in Fig. 2A und 2B dargestellt ist) und bewirkt eine stauchende Verformung der Spitze, die in ihrem distalen Endbereich am Innenschaft fixiert ist und daher dem Druck des Stempelelementes 13 nicht insgesamt nach distal ausweichen kann. Diese stauchende Verformung hat eine Aufweitung in radialer Richtung zur Folge, die aufgrund des Materials der Katheterspitze reversibel ist (elastische Aufweitung). Dieser Zustand der Katheterspitze 3 ist in Fig. 1C und 1D zu erkennen. Beim weiteren Vorschieben des Außenschaftes (Fig. 1D) wird dessen trichterartig aufgeweitetes distales Ende an die im proximalen Bereich aufgeweitete Katheterspitze 3 herangeführt, womit sich insgesamt eine "ausgebeulte", aber kantenfreie Kontur des distalen Endbereiches des Einführkatheters 1 ergibt.

In den Figuren 1A - 1D ist auf der rechten Seite, nahe dem distalen Ende des Außenschaftes 9, eine besondere konstruktive Ausgestaltung des Implantat-Konnektors 7 und, hierauf abgestimmt, des Stempelelements 13 zu sehen: Der Implantat-Konnektor 7 hat in seinem kegelstumpfförmigen proximalen Endabschnitt mehrere Durchgangslöcher 7a, durch die Streifen 13a des in jenem proximalen Endbereich streifig geschnittenen Stempelelements 13 hindurch gefädelt sind. Die Streifen 13a des Stempelelements 13 verlaufen dann noch ein Stück konzentrisch zur Längsachse des Katheters nach proximal und sind am proximalen Ende des Stempelelements mit einem Ring 13b zusammengefasst. Der Ring 13b bildet somit ein geschlossenes proximales Ende des Stempelelements 13, das mit der distalen ringförmigen Stirnfläche 9a am Außenschaft 9 in der oben skizzierten Weise zusammenwirken kann. Während der Verschiebung des Außenschaftes nach distal und der hierdurch bewirkten Bewegung des Stempelelementes in gleiche Richtung gleiten die Streifen 13a des Stempelelementes 13 durch die Durchgangslöcher 7a am Implantat-Konnektor 7.

Fig. 2A und 2B zeigen den Aufbau der Katheterspitze und des Stempelelementes in zwei Ausführungen einer Katheterspitze 3, 3' genauer. Die Katheterspitze 3 hat eine insgesamt annähernd trompetenförmige Gestalt und sitzt auf dem distalen Ende des Innenschaftes 11 des Einführkatheters. Sie ist in ihrem spitzen distalen Endbereich mit einer Klebstoffschicht 3b auf dem Innenschaft 11 fixiert, wogegen der sich aufweitenden, im Wesentlichen hohle proximale Endbereich 3c am Innenschaft nicht fixiert ist.

Wenn das Stempelelement 13 mit seinem distalen Flanschabschnitt 13a an das proximale Ende der Katheterspitze 3 drückt, wird dieses proximale Ende nach distal verschoben und zugleich der proximale Endbereich 3c der Spitze radial aufgeweitet. Diese Aufweitung ist reversibel.

Bei der in Fig. 2B gezeigten Modifikation ist am proximalen Ende der Katheterspitze 3' eine Gleitbüchse 4 eingefügt, die aus einem steiferen Kunststoffmaterial als die Katheterspitze selbst gefertigt ist, z.B. mit einer Shore-Härte von 72 oder höher, bei einer Katheterspitze mit einer Shore-Härte von 33. Die Gleitbüchse 4 verbessert einerseits die Gleitfähigkeit des proximalen spitzen Endes auf dem Innenschaft 11 und andererseits die Krafteinleitung vom Flanschabschnitt 13a des Stempelelements 13 in das proximale Ende der Katheterspitze.

Im Übrigen ist die Ausführung der Erfindung auch in einer Vielzahl von Abwandlungen der hier gezeigten Beispiele und weiter oben hervorgehobenen Aspekte der Erfindung möglich.

## Patentansprüche

1. Einführkatheter (1) zum Einführen eines Implantats an einem Zielort in einem Gefäß oder Organ eines Wirbeltiers, wobei der Einführkatheter aufweist:
einen Innenschaft (11), der zum Tragen des Implantats während des Einführens und zum Freisetzen desselben am Zielort ausgebildet ist und der eine atraumatische distale Katheterspitze (3; 3') aufweist, und
einen den Innenschaft umgebenden und diesem gegenüber verschieblichen Außenschaft (9) zum Verschieben des Implantats an den Zielort, wobei der Außenschaft an seinem distalen Ende eine Implantatkapsel (5) zur umhüllenden Aufnahme des Implantats während des Einführens aufweist,
wobei die Katheterspitze an oder nahe ihrem distalen Ende am Innenschaft fixiert und mindestens in einem proximalen Abschnitt durch eine in distaler Richtung axial wirkende Druckkraft elastisch aufweitbar ist und
proximal vom proximalen Ende der Katheterspitze ein ihr gegenüber axial verschiebliches Stempelelement (13) in den Außenschaft eingelegt, das bei einem Vorschieben des Außenschaftes relativ zum Innenschaft über einen vorbestimmten Punkt hinaus an seinem proximalen Ende mit der axial einwirkenden Druckkraft beaufschlagt wird und diese auf das proximale Ende der Katheterspitze überträgt, wodurch deren elastische Aufweitung bewirkt wird.

2. Einführkatheter nach Anspruch 1, wobei die Katheterspitze (3; 3') als mindestens im aufweitbaren proximalen Abschnitt weich-elastisches Kunststoff-Formteil ausgebildet ist.

3. Einführkatheter nach Anspruch 1 oder 2, wobei die Katheterspitze (3; 3') am proximalen Ende nicht am Innenschaft (11) fixiert, sondern beim Aufweiten gegenüber dem Innenschaft gleitbar ausgeführt ist.

4. Einführkatheter nach Anspruch 3, wobei in das proximale Ende der Katheterspitze (3; 3') eine die Gleitfähigkeit gegenüber dem Innenschaft (11) erhöhende und die Kraftübertragung vom Stempelelement auf das proximale Ende der Katheterspitze verbessernde Gleitbüchse (4) eingelegt ist.

5. Einführkatheter nach einem der vorangehenden Ansprüche, wobei die Katheterspitze (3; 3') über den größeren Teil Ihrer Längserstreckung an ihrem gesamten Innenumfang mit dem Außenumfang des Innenschaftes (11) stoffschlüssig verbunden, insbesondere verklebt oder verschweißt, ist.

6. Kathetereinrichtung nach einem der vorangehenden Ansprüche, wobei der Durchmesser der Katheterspitze (3; 3') an deren proximalem Ende im unaufgeweiteten Zustand im Wesentlichen gleich dem Durchmesser der Implantatkapsel (5) ist und der größte Durchmesser der Katheterspitze im aufgeweiteten Zustand im Bereich zwischen dem 1,05-fachen und dem 1,4-fachen, insbesondere dem 1,1-fachen und 1,35-fachen, besonders bevorzugt dem 1,25-fachen und 1,3-fachen, Durchmesser der Implantatkapsel liegt.

7. Einführkatheter nach einem der vorangehenden Ansprüche, wobei das Stempelelement (13) als in seinem distalen Bereich spiralig geschnittenes Metallrohr ausgebildet ist, an dessen distalem Ende ein Ringflansch (13a) zur Vergrößerung der Kontaktfläche mit der Katheterspitze (3; 3') ausgebildet ist.

8. Einführkatheter nach einem der vorangehenden Ansprüche, wobei am Innenschaft (11) im Inneren der Implantatkapsel ein Implantat-Konnektor (7) fest angebracht ist, der Führungsmittel (7a) zum Führen des Stempelelementes (13) bei deren axialer Verschiebung aufweist.

9. Einführkatheter nach Anspruch 8, wobei die Führungsmittel am Implantat-Konnektor (7) als Durchgangslöcher (7a) durch die Wandung des Implantat-Konnektors ausgebildet sind und das Stempelelement (13) in seinem proximalen Bereich streifig geschnitten ist, derart, dass die Streifen (13a) des Stempelelements durch die Durchgangslöcher des Implantat-Konnektors gefädelt sind, wobei die Streifen des Stempelelements an dessen proximalem Ende in einem Verbindungsring (13b) gefasst sind, der ein umfangsmäßig geschlossenes proximales Ende des Stempelelements definiert.

10. Einführkatheter nach einem der vorangehenden Ansprüche, wobei der Außenschaft (9) in seinem Verbindungsbereich mit der Implantatkapsel (7) derart aufgeweitet ist, dass eine ringförmige distale Stirnfläche (9a) gebildet ist, an die das proximale Ende des Stempelelements (13) anstößt.

11. Einführkatheter nach Anspruch 9 und 10, wobei das proximale Ende des die Metallstreifen (13a) des Stempelelements (13) zusammenfassenden Verbindungsringes (13b) an die distale ringförmige Stirnfläche (9a) des Außenschaftes anstößt.

12. Einführkatheteranordnung mit einem Einführkatheter (1) nach einem der vorangehenden Ansprüche und einem an dessen Implantatkapsel (5) angepassten kardiovaskulären Implantat, insbesondere einem Stent oder einer Herzklappenprothese.

## Claims

1. A delivery catheter (1) for inserting an implant at a target site in a vessel or organ of a mammal, wherein the delivery catheter has:
an inner shaft (11), which is designed to carry the implant during the insertion and to release the implant at the target site and which has an atraumatic distal catheter tip (3; 3'), and
an outer shaft (9), which surrounds the inner shaft and is displaceable relative thereto, for displacing the implant at the target site, wherein the outer shaft, at its distal end, carries an implant capsule (5) for receiving the implant in an encasing manner during the insertion,
wherein the catheter tip is fixed to the inner shaft at or close to the distal end thereof and, at least in a proximal portion, can be resiliently expanded by a compressive force acting axially in the distal direction, and
a plunger element (13), which, proximally from the proximal end of the catheter tip, is axially displaceable relative thereto, is inserted into the outer shaft and, as the outer shaft is moved forward relative to the inner shaft beyond a predetermined point, is acted on at its proximal end by the axially acting compressive force and transfers this to the proximal end of the catheter tip, whereby the resilient expansion thereof is implemented.

2. The delivery catheter according to claim 1, wherein the catheter tip (3; 3') is formed as a soft-resilient moulded plastics part, at least in the expandable proximal portion.

3. The delivery catheter according to claim 1 or 2, wherein the catheter tip (3; 3') is not fixed to the inner shaft (11) at the proximal end, but can be slid relative to the inner shaft when expanded.

4. The delivery catheter according to claim 3, wherein a plain bush (4), which increases the slidability relative to the inner shaft (11) and improves the transfer of force from the plunger element to the proximal end of the catheter tip, is inserted into the proximal end of the catheter tip (3; 3').

5. The delivery catheter according to any one of the preceding claims, wherein the catheter tip (3; 3') is connected over the greater part of its longitudinal extent at its entire inner periphery to the outer periphery of the inner shaft (11) in an integrally bonded manner, in particular is glued or welded.

6. The delivery catheter according to any one of the preceding claims, wherein the diameter of the catheter tip (3; 3'), at the proximal end thereof in the unexpanded state, is substantially equal to the diameter of the implant capsule (5), and the greatest diameter of the catheter tip in the expanded state lies in a range between 1.05 times and 1.4 times, in particular 1.1 times and 1.35 times, particularly preferably 1.25 times and 1.3 times, the diameter of the implant capsule.

7. The delivery catheter according to any one of the preceding claims, wherein the plunger element (13) is formed as a spirally cut metal tube in its distal region, at the distal end of which there is formed a ring flange (13a) for enlarging the area of contact with the catheter tip (3; 3').

8. The delivery catheter according to any one of the preceding claims, wherein an implant connector (7) is fixedly connected to the inner shaft (11) inside the implant capsule and has guide means (7a) for guiding the plunger element (13) during the axial displacement thereof.

9. The delivery catheter according to claim 8, wherein the guide means at the implant connector (7) are formed as through-holes (7a) through the wall of the implant connector and the plunger element (13) is cut in a strip-like manner in its proximal region, in such a way that the strips (13a) of the plunger element are threaded through the through-holes of the implant connector, wherein the strips of the plunger element are gripped at the proximal end thereof in a connection ring (13b), which defines a peripherally closed proximal end of the plunger element.

10. The delivery catheter according to any one of the preceding claims, wherein the outer shaft (9) is expanded in its region of connection to the implant capsule (7) in such a way that an annular distal end face (9a) is formed, which is contacted by the proximal end of the plunger element (13).

11. The delivery catheter according to claim 9 and 10, wherein the proximal end of the connection ring (13b) combining the metal strips (13a) of the plunger element (13) contacts the distal annular end face (9a) of the outer shaft.

12. A delivery catheter arrangement comprising a delivery catheter (1) according to any one of the preceding claims and a cardiovascular implant, in particular a stent or a heart valve prosthesis, adapted to the implant capsule (5) of said delivery catheter.

## Revendications

1. Cathéter d'insertion (1) destiné à l'insertion d'un implant à un endroit cible dans un vaisseau ou un organe d'un vertébré, où le cathéter d'insertion présente:
une tige intérieure (11), qui est prévue pour porter l'implant pendant l'insertion et pour le libérer à l'endroit cible, et qui présente une pointe de cathéter (3; 3') distale non traumatisante; et
une tige extérieure (9), entourant la tige intérieure et étant déplaçable par-rapport à celle-ci, pour le déplacement de l'implant à l'endroit cible, où la tige extérieure présente à son extrémité distale une capsule d'implant (5) pour la réception enveloppante de l'implant durant l'insertion,
où la pointe de cathéter est fixée sur la tige intérieure au niveau ou près de son extrémité distale, et peut être élargie de manière élastique au moins dans un segment proximal par une force de compression agissant axialement dans la direction distraie, et
du côté proximale par rapport à l'extrémité proximale de la pointe de cathéter, un élément de poinçon (13), déplaçable axialement par rapport à celle-ci, est inséré dans la tige extérieure, celui-ci étant soumis à la force de compression agissant axialement à son extrémité proximale lors d'une avancée de la tige extérieure par rapport à la tige intérieure au-delà d'un point prédéfini et transmettant celle-ci à l'extrémité proximale de la pointe de cathéter, provoquant ainsi son élargissement élastique.

2. Cathéter d'insertion selon la revendication 1, dans lequel la pointe de cathéter (3; 3') est conçue au moins sous forme d'une pièce moulée en matière plastique élastique et souple dans le segment proximal pouvant être élargi.

3. Cathéter d'insertion selon la revendication 1 ou 2, dans lequel la pointe de cathéter (3; 3') ne se fixe pas à la tige intérieure (11) au niveau de l'extrémité proximale, mais est conçue de manière à glisser par rapport à la tige intérieure lors de l'élargissement.

4. Cathéter d'insertion selon la revendication 3, dans lequel un manchon de glissement (4), augmentant le glissement par rapport à la tige intérieure (11) et améliorant la transmission de la force de l'élément de poinçon sur l'extrémité proximale de la pointe de cathéter est inséré dans l'extrémité proximale de la pointe de cathéter (3; 3').

5. Cathéter d'insertion selon l'une des revendications précédentes, dans lequel la pointe de cathéter (3; 3') est reliée par complémentarité des matières, notamment collée ou soudée, avec le pourtour extérieur de la tige intérieure (11) au niveau de son pourtour intérieur complet sur la plus grande partie de son extension longitudinale.

6. Cathéter d'insertion selon l'une des revendications précédentes, dans lequel le diamètre de la pointe de cathéter (3; 3') à son extrémité proximale, dans l'état non élargi, est sensiblement égal au diamètre de la capsule d'implant (5), et le diamètre le plus grand de la pointe de cathéter à l'état élargi se situe dans la plage entre 1,05 fois et 1,4 fois, notamment entre 1,1 fois et 1,35 fois, de manière particulièrement préférée entre 1,25 fois et 1,3 fois, le diamètre de la capsule d'implant.

7. Cathéter d'insertion selon l'une des revendications précédentes, dans lequel l'élément de poinçon (13) est conçu, dans sa région distale, sous la forme d'un tube métallique taillé en spirale à l'extrémité duquel est formé un rebord annulaire (13a) destiné à l'agrandissement de la surface de contact avec la pointe de cathéter (3; 3').

8. Cathéter d'insertion selon l'une des revendications précédentes, dans lequel un connecteur d'implant (7), qui présente des moyens de guidage (7a) pour le guidage de l'élément de poinçon (13) lors de son déplacement axial, est fermement attaché sur la tige intérieure (11) à l'intérieur de la capsule d'implant.

9. Cathéter d'insertion selon la revendication 8, dans lequel les moyens de guidage sont formés sur le connecteur d'implant (7) sous forme de trous traversants (7a) la paroi du connecteur d'implant, et l'élément de poinçon (13) est taillé avec des rayures dans sa région proximale, de telle manière que les rayures (13a) de l'élément de poinçon sont filetées par les trous traversants du connecteur d'implant, où les rayures de l'élément de poinçon sont rassemblées à son extrémité proximale dans un anneau de logement (13b) qui définit une extrémité proximale fermée de l'élément de poinçon.

10. Cathéter d'insertion selon l'une des revendications précédentes, dans lequel la tige extérieure (9) est élargie au niveau de sa zone de liaison avec la capsule d'implant (7) de telle manière qu'une surface frontale (9a) distale de forme annulaire est formée, sur laquelle s'appuie l'extrémité proximale de l'élément de poinçon (13).

11. Cathéter d'insertion selon la revendication 9 ou 10, dans lequel l'extrémité proximale de l'anneau de liaison (13b) rassemblant les rayures (13a) métalliques de l'élément de poinçon (13) s'appuie sur la surface frontale (9a) distale en forme d'anneau de la tige extérieure.

12. Agencement de cathéter d'insertion doté d'un cathéter d'insertion (1) selon l'une des revendications précédentes et d'un implant cardiovasculaire adapté à sa capsule d'implant (5), notamment un stent ou une prothèse de valve cardiaque.
